# EUROPEAN PATENT SPECIFICATION

(11) **EP 4 452 084 B1**
(45) Date of publication and mention of the grant of the patent: **03.06.2026**
(21) Application number: 22840647.6
(22) Date of filing: 20.12.2022
(51) Int. Cl.: A61B 8/08, A61B 8/00

(54) **ADAPTIVE DUAL FOCUS LUNG FEATURE VISUALIZATION IN ULTRASOUND IMAGING**
ADAPTIVE DOPPELFOKUS-LUNGENFUNKTIONSVISUALISIERUNG IN DER ULTRASCHALLBILDGEBUNG
VISUALISATION ADAPTATIVE DE CARACTÉRISTIQUES PULMONAIRES À DOUBLE FOYER DANS UNE IMAGERIE ULTRASONORE

(30) Priority: 21.12.2021 US 202163292169 P
(43) Date of publication of application: 30.10.2024
(73) Proprietor: Koninklijke Philips N.V., 5656 AE Eindhoven (NL)
(72) Inventor: ERKAMP, Ramon Quido, 5656 AG Eindhoven (NL); GADES, Anthony M., 5656 AG Eindhoven (NL); POLAND, McKee Dunn, 5656 AG Eindhoven (NL); RAJU, Balasundar Iyyavu, 5656 AG Eindhoven (NL); HUNT, Thomas James, 5656AG Eindhoven (NL)
(74) Representative: Philips Intellectual Property & Standards
(86) International application number: PCT/EP2022/086828
(87) International publication number: WO 2023/118029

(56) References cited:
- US-A1- 2020 046 314
- US-A1- 2020 359 991
- LIU JING ET AL: "Specification and guideline for technical aspects and scanning parameter settings of neonatal lung ultrasound examination", JOURNAL OF MATERNAL-FETAL AND NEONATAL MEDICINE, vol. 35, no. 5, 28 June 2021 (2021-06-28), UK, pages 1003 - 1016, XP093033822, ISSN: 1476-7058, DOI: 10.1080/14767058.2021.1940943
- MOSHAVEGH RAMIN ET AL: "Novel automatic detection of pleura and B-lines (comet-tail artifacts) on in vivo lung ultrasound scans", PROGRESS IN BIOMEDICAL OPTICS AND IMAGING, SPIE - INTERNATIONAL SOCIETY FOR OPTICAL ENGINEERING, BELLINGHAM, WA, US, vol. 9790, 1 April 2016 (2016-04-01), pages 97900K - 97900K, XP060065636, ISSN: 1605-7422, ISBN: 978-1-5106-0027-0, DOI: 10.1117/12.2216499
- KAMEDA TORU ET AL: "The Mechanisms Underlying Vertical Artifacts in Lung Ultrasound and Their Proper Utilization for the Evaluation of Cardiogenic Pulmonary Edema", DIAGNOSTICS, vol. 12, no. 2, 20 January 2022 (2022-01-20), pages 252, XP093033783, DOI: 10.3390/diagnostics12020252

## Description

### TECHNICAL FIELD

The present disclosure relates generally to ultrasound systems and methods for obtaining ultrasound images of an anatomy. For example, an ultrasound system can suppress or enhance imaging artifacts in a lung image obtained by an ultrasound imaging device.

### BACKGROUND

Ultrasound imaging is frequently used to obtain images of internal anatomical structures of a patient. Ultrasound systems typically comprise an ultrasound transducer probe that includes one or more ultrasound transducer elements. An ultrasound transducer element is activated to vibrate at ultrasonic frequencies to transmit ultrasonic energy into the patient's anatomy, and then receive ultrasonic echoes reflected or backscattered by the patient's anatomy to create an image.

Ultrasound probes can be used to diagnose lung conditions such as consolidation, pleural effusion, air bronchogram, fluid, or pneumothorax. However, ultrasound images that include an air-tissue interface, where the density of the sound-carrying medium changes abruptly, can be prone to imaging artifacts such as (generally horizontal) A-line artifacts and (generally vertical) B-line artifacts. This is true for example at the pleural line of a lung, where tissue is in direct contact with inhaled air stored within the lung. In some instances, image artifacts may be indicative of a health condition of the patient's lung tissue, and thus may be clinically useful. However, in other cases, image artifacts may obscure true anatomy and thus interfere with proper diagnosis.

Both artifacts and true anatomy can be important in lung imaging, and if a feature is seen in a region of interest (e.g., below the pleural line), it may be difficult to discern whether the feature is from true anatomy at depth or originates from an artifact generated in the shallower pleural line. In conventional imaging, the ultrasound system may use dynamic receive beamforming where the focal depth is the same as the imaging depth over the full depth range, and for transmit beamforming multiple depth regions can be formed each with its own transmit focus centered for that region.

In lung ultrasound it is sometimes the true anatomical feature that is more important (such as in consolidation), and sometimes it is an imaging artifact (e.g., A-lines and/or B-lines) that is more important. Moreover, due to artifacts in lung imaging there is sometimes ambiguity as to whether a feature observed in the image originates from true anatomy at the observed location or originates from anatomy in another location generating artifacts.

In order to obtain clear images of imaging artifacts below the pleural line, it may be desirable to set the focal depth of the ultrasound transmission beam to the depth of the pleural line. However, this increases the difficulty of visualizing the lung tissue beneath the pleural line in clinically relevant detail.

"Specification and guideline for technical aspects and scanning parameter settings of neonatal lung ultrasound examination" by Lui Jing et al., Journal of Maternal-Fetal and Neonatal Medicine, vol. 35, no. 5, 28 June 2021, pp. 1003-1016 (XP093033822) discloses an instrument operation specification and parameter setting guidelines for neonatal lung ultrasound.

"Novel Automatic Detection of Pleura and B-lines (Comet-Tail Artifacts) on In-Vivo Lung Ultrasound Scans" by Moshavegh Ramin et al., Progress in Biomedical Optics and Imaging, SPIE - International Society for Optical Engineering, Bellingham, WA, US, vol. 9790, 1 April 2016, pp. 97900K-97900K (XP060065636), discloses a novel automatic method for detection of B-lines (comet-tail artifacts) in lung ultrasound scans.

### SUMMARY

The invention is defined by the independent claims. The dependent claims define advantageous embodiments. The present disclosure provides systems, devices, and methods for dual-focus imaging of anatomical structures such as lung tissue. This can be done for example to enhance the appearance of an anatomical structure such as the pleural line, while suppressing imaging artifacts that may result from such enhancement, or to enhance image artifacts while reducing the appearance of true anatomical features.

The present disclosure teaches the use of two imaging frames, one optimized for artifact enhancement, and one optimized for true anatomy enhancement. Information in both these frames is used to enhance appearance of a feature of interest in a region of interest (ROI). If the feature of interest in the ROI is believed to be an artifact, then the frame optimized for artifact enhancement may be more heavily used, whereas if the feature is believed to be true anatomy, the true anatomy enhancement frame is more heavily used.

Image artifacts may for example include A-line artifacts and B-line artifacts. Image artifacts may be indicative of a health condition, but may also obscure true anatomy. Focusing both the transmit beam and the receive beam of the ultrasound system on specific tissue features (e.g., the pleural line) may also enhance artifacts, even if such enhancement of artifacts is not desired. Conversely, focusing the transmit beam and receive beam on tissue of interest beneath the pleural line may enhance the appearance of the tissue of interest, while decreasing the appearance of the pleural line and the image artifacts. The present disclosure also includes constructing a combined image that includes features captured at two or more focal depths, such that both the pleural line and the tissue of interest may be captured clearly.

The present disclosure deviates from conventional imaging by, for example, using a focal depth at the pleural line while imaging at the deeper ROI, and showing this enhanced artifact view when the feature in the ROI is believed to be, or originate from, an artifact.

A system of one or more computers / processors can be configured to perform particular operations or actions by virtue of having software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions. One general aspect includes a system for imaging anatomy of a patient. The system includes an ultrasound probe configured to obtain ultrasound imaging data representative of the anatomy; and a processor communicatively coupled to the ultrasound probe. The processor is configured to: generate, using the ultrasound imaging data received from the ultrasound probe, an initial image including an anatomical feature, an image artifact caused by the anatomical feature, and a region of interest disposed below the anatomical feature and obscured by the image artifact; and determine a depth of the anatomical feature and a depth of the region of interest. The processor is further configured to generate, using the ultrasound imaging data received from the ultrasound probe, a first image including the anatomical feature, the region of interest, and the image artifact, where a focal depth of a transmit beam associated with the first image and a focal depth of a receive beam associated with the first image are set to the depth of the anatomical feature, where an appearance of the anatomical feature and the image artifact in the first image is enhanced with respect to the initial image, and where an appearance of the region of interest in the first image is decreased with respect to the initial image. The processor is further configured to generate, using the ultrasound imaging data received from the ultrasound probe, a second image including the anatomical feature, the region of interest, and the image artifact, where the focal depth of the transmit beam associated with the second image and the focal depth of the receive beam associated with the second image are set to the depth of the region of interest, where the appearance of the anatomical feature and the image artifact in the second image is decreased with respect to the initial image, and where the appearance of the region of interest in the second image is enhanced with respect to the initial image; and provide, to a display in communication with the processor, an output based on at least one of the first image or the second image. Other embodiments of this aspect include corresponding computer systems, apparatus, and computer programs recorded on one or more computer storage devices, each configured to perform the actions of the methods.

Implementations may include one or more of the following features. In some embodiments, the output includes the first image and the second image. In some embodiments, the first image and the second image are displayed simultaneously. In some embodiments, the processor is further configured to generate a combined image including aspects of the first image and the second image, where the output includes the combined image. In some embodiments, portions of the combined image are assigned a color based on whether the portions include the anatomical feature or the imaging artifact. In some embodiments, the anatomy includes a lung. In some embodiments, the anatomical feature includes a pleural line of the lung. In some embodiments, the processor is further configured to automatically identify the pleural line. In some embodiments, the processor is further configured to determine, based on the first image and the second image, whether the region of interest contains a feature of interest. In some embodiments, the feature of interest includes at least one of a consolidation, pleural effusion, air bronchogram, fluid, or pneumothorax. In some embodiments, the processor is further configured to automatically identify the feature of interest. In some embodiments, the image artifact includes one or more A-line artifacts. In some embodiments, the image artifact includes one or more B-line artifacts. In some embodiments, the processor is configured to: receive a user input, from the user interface device, identifying the region of interest in the initial image. Implementations of the described techniques may include hardware, a method or process, or computer software on a computer-accessible medium.

One general aspect includes a method for imaging anatomy of a patient. The method includes generating, with a processor communicatively coupled to an ultrasound probe, an initial image using ultrasound imaging data received from the ultrasound probe, where the initial image includes an anatomical feature, an image artifact caused by the anatomical feature, and a region of interest disposed below the anatomical feature and obscured by the image artifact; and determining, with the processor, a depth of the anatomical feature and a depth of the region of interest. The method also includes generating, with the processor, a first image using the ultrasound imaging data received from the ultrasound probe, where the first image includes the anatomical feature, the region of interest, and the image artifact, where a focal depth of a transmit beam associated with the first image and a focal depth of a receive beam associated with the first image are set to the depth of the anatomical feature, where an appearance of the anatomical feature and the image artifact in the first image is enhanced with respect to the initial image, and where an appearance of the region of interest in the first image is decreased with respect to the initial image. The method also includes generating, with the processor, a second image using the ultrasound imaging data received from the ultrasound probe, where the second image includes the anatomical feature, the region of interest, and the image artifact, where the focal depth of the transmit beam associated with the second image and the focal depth of the receive beam associated with the second image are set to the depth of the region of interest, where the appearance of the anatomical feature and the image artifact in the second image is decreased with respect to the initial image, and where the appearance of the region of interest in the second image is enhanced with respect to the initial image. The method also includes providing, to a display in communication with the processor, an output based on at least one of the first image or the second image. Other embodiments of this aspect include corresponding computer systems, apparatus, and computer programs recorded on one or more computer storage devices, each configured to perform the actions of the methods.

Additional aspects, features, and advantages of the present disclosure will become apparent from the following detailed description.

### BRIEF DESCRIPTION OF THE DRAWINGS

Illustrative embodiments of the present disclosure will be described with reference to the accompanying drawings, of which:
**Figure 1** is a diagrammatic schematic view of an ultrasound imaging system.
**Figures 2A and 2B** are schematic representations of example lung ultrasound images.
**Figures 3A and 3B** are schematic representations of example lung ultrasound images including depictions of transmit beams.
**Figures 4A and 4B** are schematic representations of example lung ultrasound images including depictions of receive beams.
**Figures 5A and 5B** **are** schematic representations of example lung ultrasound images including depictions of transmit beams and receive beams.
**Figures 6A, 6B****,** **7A** and **7B** **are** schematic representations of example lung ultrasound images.
**Figure 8** **and** **9** **are** schematic representations of example combined lung ultrasound images.
**Figure 10A and 10** **B** are a schematic representation of an example lung ultrasound image comprising B-line artefacts.
**Figure 11** shows a flow diagram of an example dual-focus ultrasound imaging method.
**Figure 12** is a schematic diagram of a processor circuit.

### DETAILED DESCRIPTION

The present disclosure provides systems and methods for dual-focus imaging of anatomical structures such as lung tissue. This can be done for example to enhance the appearance of an anatomical structure such as the pleural line (as practiced in conventional imaging with multiple transmit focal zones and dynamic receive beamforming), while suppressing imaging artifacts that may result from such enhancement, and/or to enhance the appearance of artifacts while reducing the appearance of true anatomical features (a deviation from conventional imaging systems and methods).

Image artifacts may for example include A-line artifacts and B-line artifacts, as described below. In some instances, image artifacts may be indicative of a health condition of the patient's lung tissue, and thus a caregiver may wish to enhance the artifacts in order to gain information about the health condition. In other instances, a caregiver may wish to enhance specific tissue features (e.g., features of the pleural line) that give rise to the artifacts, which may have the effect of enhancing artifacts even if such enhancement of artifacts is not necessary or desired.

If the pleural line does create artifacts, it may be clinically useful or desirable for the imaging system to enhance them where they are believed to have diagnostic value. Thus, in a region of interest (ROI) below the pleural line, the imaging system may still set the focal depth to the depth of the pleural line, so the artifacts in the ROI are enhanced. However, if there is true anatomy at the ROI, the clinician may want the imaging system to enhance the anatomy while reducing the artifacts. In order to visualize the pleural line clearly while minimizing the artifacts below it, the imaging system may for example use conventional imaging with one transmit focus on the pleural line and another one deeper (e.g., at the ROI), while employing dynamic receive beamforming. However, in the process the clinician may lose valuable diagnostic information that the artifact in the ROI would show.

Thus, it is desirable to show both enhanced artifacts and enhanced true anatomy. This may be done for example by showing both enhanced images side by side, or through various kinds of smart blending. Many nuanced implementation options exist for smart blending of the two images, and fall within the scope of the present disclosure. In a non-limiting example, a B-line extending from the pleural line to beyond the depth of the ROI would likely block out true anatomy, so the anatomy-enhanced image may be used to study the ROI. In another non-limiting example, a group of A-lines may extend to a fairly modest depth, so where an A-line falls in ROI, the imaging system may use the anatomy-enhanced image (or portions thereof) to show the ROI itself, but use the artifact-enhanced image for the spaces right below and above the ROI. Other smart blending techniques, procedures, or algorithms may be used instead or in addition, including algorithms that automatically detect or identify anatomical features and/or image artifacts.

Selective enhancement of image artifacts may be achievable, for example, by setting the focal depth of both the transmit beam and the receive beam to the depth of the pleural line. In other instances, image artifacts may obscure actual tissue (including diseased tissue in a region of interest), and thus a practitioner may wish to suppress the artifacts and enhance the appearance of tissue within the region of interest. This may be achievable for example by setting the focal depth of both the transmit beam and the receive beam to the depth of the region of interest.

A comparison between images captured at two different focal depths may reveal, for example, whether a brightness observed at the depth of interest is caused by real anatomy or an imaging artifact. Accordingly, it may then be desirable to construct a combined image that includes portions of a first image, captured at a first focal depth, and containing a first anatomical feature (e.g., the pleural line) as well as portions of a second image, captured at a second focal depth, and containing a second anatomical feature (e.g., the tissue within the region of interest). Such a combined image may be particularly useful to ultrasound technicians in capturing high-quality diagnostic information of lung features at different depths, and to diagnosticians and other caregivers in determining the health status of lung tissue at different depths. This approach may for example be desirable when there is real anatomy at depth of interest, as may often be the case in conventional imaging. Conversely, in cases where a feature of interest is suspected to be an artifact, then the ROI portion of the combined image may be taken from the artifact-enhanced image, while other portions are taken from the anatomy-enhanced image. This may be at odds with conventional imaging algorithms, which would not normally employ shallow transmit and receive focusing for deeper depth.

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

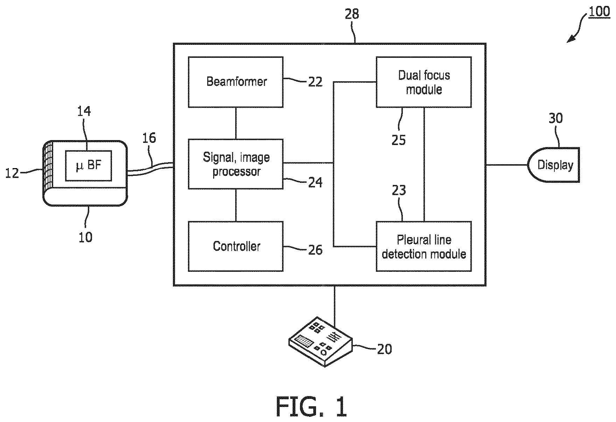

**Figure 1** is a diagrammatic, block diagram view of an ultrasound system 100 according to at least one embodiment of the present disclosure. An ultrasound probe 10 has a transducer array 12 comprising a plurality of ultrasound transducer elements. In some instances, the array 12 may include any number of ultrasound transducer elements. For example, the array 12 can include between 1 transducer element and 1000 transducer elements, including values such as 2 transducer elements, 4 transducer elements, 36 transducer elements, 64 transducer elements, 128 transducer elements, 500 transducer elements, 812 transducer elements, and/or other values both larger and smaller. In some instances, the transducer elements of the array 12 may be arranged in any suitable configuration, such as a linear array, a planar array, a curved array, a curvilinear array, a circumferential array, an annular array, a phased array, a matrix array, a one-dimensional (lD) array, a 1.x dimensional array (e.g., a 1.5D array), or a two-dimensional (2D) array. The array of transducer elements (e.g., one or more rows, one or more columns, and/or one or more orientations) can be uniformly or independently controlled and activated. The array 12 can be configured to obtain one-dimensional, two-dimensional, and/or three-dimensional images of patient anatomy.

The ultrasound transducer elements may comprise piezoelectric/piezoresistive elements, piezoelectric micromachined ultrasound transducer (PMUT) elements, capacitive micromachined ultrasound transducer (CMUT) elements, and/or any other suitable type of ultrasound transducer elements. The ultrasound transducer elements of the array 12 are in communication with (e.g., electrically coupled to) electronic circuitry 14. In some embodiments, such as the embodiment of Fig. 1, the electronic circuitry 14 can comprise a microbeamformer (µBF). In other embodiments, the electronic circuitry comprises a multiplexer circuit (MUX). The electronic circuitry 14 is located in the probe 10 and communicatively coupled to the transducer array 12. In some embodiments, one or more components of the electronic circuitry 14 can be positioned in the probe 10. In some embodiments, one or more components of the electronic circuitry 14, can be positioned in a processor 28, or processing system. In some aspects, some components of the electronic circuitry 14 are positioned in the probe 10 and other components of the electronic circuitry 14 are positioned in the processor 28. The electronic circuitry 14 may comprise one or more electrical switches, transistors, programmable logic devices, or other electronic components configured to combine and/or continuously switch between a plurality of inputs to transmit signals from each of the plurality of inputs across one or more common communication channels. The electronic circuitry 14 may be coupled to elements of the array 12 by a plurality of communication channels. The electronic circuitry 14 is coupled to a cable 16, which transmits signals including ultrasound imaging data to the processor 28.

In the processor 28, the signals are digitized and coupled to channels of a system beamformer 22, which appropriately delays each signal. The delayed signals are then combined to form a coherent steered and focused receive beam. System beamformers may comprise electronic hardware components, hardware controlled by software, or a microprocessor executing beamforming algorithms. In that regard, the beamformer 22 may be referenced as electronic circuitry. In some embodiments, the beamformer 22 can be a system beamformer, such as the system beamformer 22 of Fig. 1, or it may be a beamformer implemented by circuitry within the ultrasound probe 10. In some embodiments, the system beamformer 22 works in conjunction with a microbeamformer (e.g., electronic circuitry 14) disposed within the probe 10. The beamformer 22 can be an analog beamformer in some embodiments, or a digital beamformer in some embodiments. In the case of a digital beamformer, the system includes A/D converters which convert analog signals from the array 12 into sampled digital echo data. The beamformer 22 generally will include one or more microprocessors, shift registers, and or digital or analog memories to process the echo data into coherent echo signal data. Delays are effected by various means such as by the time of sampling of received signals, the write/read interval of data temporarily stored in memory, or by the length or clock rate of a shift register as described in US4173007. Additionally, in some embodiments, the beamformer can apply appropriate weight to each of the signals generated by the array 12. The beamformed signals from the image field are processed by a signal and image processor 24 to produce 2D or 3D images for display on an image display 30. The signal and image processor 24 may comprise electronic hardware components, hardware controlled by software, or a microprocessor executing image processing algorithms. It generally will also include specialized hardware or software which processes received echo data into image data for images of a desired display format such as a scan converter. In some embodiments, beamforming functions can be divided between different beamforming components. For example, in some embodiments, the system 100 can include a microbeamformer located within the probe 10 and in communication with the system beamformer 22. The microbeamformer may perform preliminary beamforming and/or signal processing that can reduce the number of communication channels required to transmit the receive signals to the processor 28.

Control of ultrasound system parameters such as scanning mode (e.g., B-mode, M-mode), probe selection, beam steering and focusing, and signal and image processing is done under control of a system controller 26 which is coupled to various modules of the system 100. The system controller 26 may be formed by application specific integrated circuits (ASICs) or microprocessor circuitry and software data storage devices such as random-access memories (RAMs), read only memories (ROMs), or disk drives. In the case of the probe 10, some of this control information may be provided to the electronic circuitry 14 from the processor 28 over the cable 16, conditioning the electronic circuitry 14 for operation of the array as required for the particular scanning procedure. The user inputs these operating parameters by means of a user interface device 20.

In some embodiments, the image processor 24 is configured to generate images of different modes to be further analyzed or output to the display 30. For example, in some embodiments, the image processor can be configured to compile a B-mode image, such as a live B-mode image, of an anatomy of the patient. In other embodiments, the image processor 24 is configured to generate or compile an M-mode image. An M-mode image can be described as an image showing temporal changes in the imaged anatomy along a single scan line. The M-mode image can comprise a plurality of samples, each sample including a line of ultrasound imaging data obtained at a particular time. In that regard, the M-mode image shows ultrasound imaging data obtained along the scan line over a period of time, with each sample obtained at a different time. For example, an M-mode image can show a plurality of samples along the x-axis, where each sample shows an intensity or amplitude as a function of depth on the y-axis, the amplitude represented by varying shades applied to each pixel of depth along the scan line. In some embodiments, the probe 10 can be controlled by the processor to operate in an M-mode to obtain the M-mode image. In other embodiments, the M-mode image can be compiled, reconstructed, or generated using a plurality of B-mode image frames. In some embodiments, the M-mode image includes ultrasound imaging data obtained for at least one heart cycle. In some embodiments, the M-mode image includes ultrasound imaging data obtained for several heart cycles, such as 5, 10, 15, or more heart cycles.

The processor 28 includes a pleural line detection module 23 and a dual focus module 25. The pleural line detection module can be configured to analyze processed ultrasound imaging data from the image processor 24 to identify, or detect, a pleural line in the image. The pleural line detection module can identify the pleural line in a B-mode image and/or an M-mode image. In an exemplary embodiment, as will be further described below, the pleural line detection module is configured to identify both the presence and location of a pleural line in an M-mode image. For example, WO2017/162860 provides exemplary systems, devices, and methods for the automated detection of pleural lines.

The dual focus module 25 is configured to adjust focal depth in order to enhance either image artifacts or actual tissue structures at a given depth. Image artifacts may be indicative of a health condition of the patient's lung tissue, and thus a caregiver may wish to enhance the artifacts, or specific tissue features that give rise to the artifacts, by setting a focal depth of the ultrasound system 100 to, for example, the depth of the pleural line. In other instances, image artifacts may obscure actual tissue in a region of interest, and thus a practitioner may wish to suppress the artifacts and enhance the appearance of the actual tissue, by aligning the focal depth to the region of interest. The dual focus module 25 may also construct a combined ultrasound image that includes, for example, both an enhanced pleural line from a first ultrasound image and an enhanced region of interest from a second image. If the ROI contains a feature that originates from true anatomy, then an anatomy-enhanced image may be used for the ROI, whereas if the ROI contains a feature that originates from one or more artifacts, then an artifact-enhanced image may be used for the ROI.

It will be understood that various components of the processor 28, such as the pleural line detection module 23 and/or the dual focus module 25, can be carried out by a computing device, such as a computer processor in communication with a computer-readable memory device comprising computer-readable instructions to carry out the functions of the various components of the processor 28. Thus, the processor 28 can comprise hardware, such as a computer processor, application-specific integrated circuit (ASIC), field-programmable gate array (FPGA), capacitors, resistors, and/or other electronic devices, software, or a combination of hardware and software. In some embodiments, each component or module of the processor 28 is performed by a single computing device. In other embodiments, the various components of the processor 28 (e.g., modules 23, 25, beamformer 22, signal and image processor 24, etc.) are performed by separate computer devices in communication with one another.

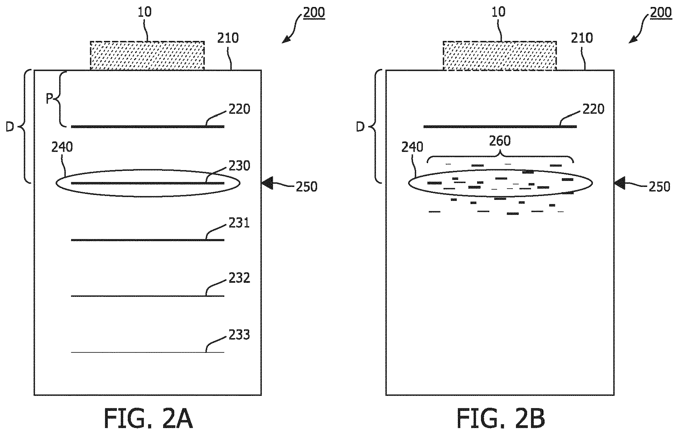

**Figure 2A** is a schematic representation of an example lung ultrasound image 200. The ultrasound probe 10 is pressed against a body surface 210 of a patient, and produces an ultrasound image 200 that includes the pleural line 220, which may for example be an interface between lung tissue and an air volume held within the lung. The pleural line may for example appear as an echogenic horizontal line, located approximately a half-centimeter deeper than the shallowest extent of the ribs. The pleural line consists of the closely opposed visceral and parietal pleura. In a normal lung, the visceral pleura can be seen sliding back and forth against the parietal pleura producing a physiological phenomenon called "lung sliding", with a glistening or shimmering appearance as the subject breathes.

Also visible is an A-line artifact 230, which may for example be an echo or harmonic of the pleural line located at, for example, 2-times (2X) the pleural depth P (e.g., the distance between the body surface 210 and the pleural line 220). Additional A-line artifacts 231, 232, and 233 are also visible, located for example at 3X, 4X, and 5X the pleural depth P. In an example, the A-line artifacts grow fainter with depth, such that A-line artifact 230 is fainter than pleural line 220, A-line artifact 231 is fainter than A-line artifact 230, A-line artifact 232 is fainter than A-line artifact 231, and A-line artifact 233 is fainter than A-line artifact 232.

In some examples, the presence, shape, or brightness of A-line artifacts may be evidence of a health condition of the pleural line 220. For example, bright A-lines 230, 231, 232, and 233 may be indicative of a healthy pleural line 220, whereas dim, diffuse, or irregular A-line artifacts 230, 231, 232, and 233 may be indicative of pleural line anomalies of interest to caregivers.

Also visible is a depth selector of focal depth selector 250, indicating a desired focal depth D for the ultrasound image 200. The depth selector 250 is centered on a region of interest 240, which may for example be a suspected location of lung tissue to be clinically evaluated. However, A-line artifact 230 falls within region of interest 240, which may make anatomical features within the region of interest difficult to image clearly. Thus, it is understood that A-lines may be important in determining the health of the pleural line 220, but may also introduce difficulties in imaging a region of interest.

It should be noted that the focal depth of an ultrasound image may be selected by myriad different methods, including but not limited to physical controls (buttons, sliders, dials, switches, etc.) or virtual controls (e.g., touchscreen controls, mouse controls, etc.), and may or may not be accompanied by a visual indicator such as focal depth selector 250. As such, focal depth selector 250 should be considered exemplary rather than limiting, and is included herein at least partly for explanatory purposes, to show where the focal depth is set for the ultrasound images depicted herein.

**Figure 2B** is a schematic representation of an example lung ultrasound image 200. Visible are the ultrasound probe 10, body surface 210, pleural line 220, region of interest 240, and depth selector 250. Within the region of interest 240, or partially overlapping with the region of interest 240, is an anatomical feature 260. The anatomical feature 260 may for example be healthy tissue, or may be a consolidation, pleural effusion, air bronchogram, fluid, pneumothorax, or another lung anomaly.

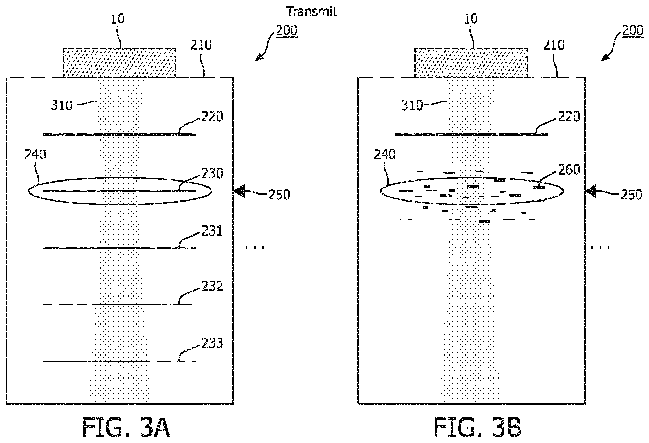

**Figure 3A** is a schematic representation of an example lung ultrasound image 200. Visible are the ultrasound probe 10, body surface 210, pleural line 220, region of interest 240, depth selector 250, and A-line artifacts 230, 231, 232, and 233. Also visible is an ultrasound transmit beam or transmission beam 310. In the example shown in Figure 3A, the ultrasound transmission beam 310 has an hourglass shape, with the narrowest portion defining and coinciding with the focal depth of the ultrasound image 200, which in this case is set to the depth of the region of interest 240, which also happens to coincide with the depth of an A-line artifact 230.

**Figure 3B** is a schematic representation of an example lung ultrasound image 200. Visible are the ultrasound probe 10, body surface 210, pleural line 220, region of interest 240, depth selector 250, anatomical feature 260, and ultrasound transmission beam 310. In the example shown in Figure 3B, the narrowest point of the transmission beam 310 (e.g., the focal depth of the ultrasound image 200) coincides with the anatomical feature 260, which may for example have the effect of focusing the anatomical feature 260 in the ultrasound image 200, while defocusing the pleural line 220 to some extent.

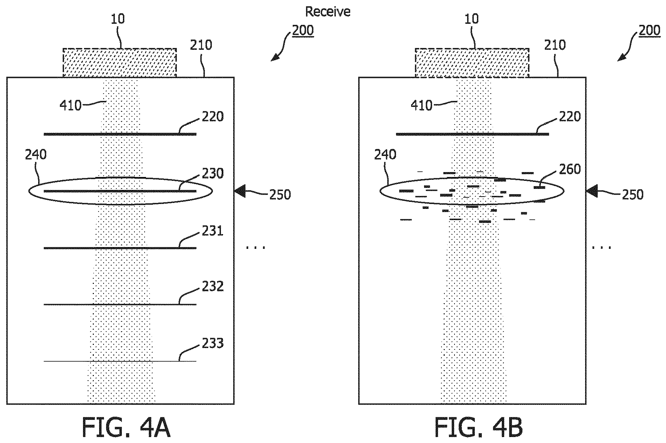

**Figure 4A** is a schematic representation of an example lung ultrasound image 200. Visible are the ultrasound probe 10, body surface 210, pleural line 220, region of interest 240, depth selector 250, and A-line artifacts 230, 231, 232, and 233. Also visible is an ultrasound reception beam or receive beam 410, which in traditional ultrasound imaging may also be described for example as a viewing cone. The receive beam 410 defines a region of space from which ultrasound echoes (e.g., echoes of the transmit beam 310, as seen for example in Figures 3A and 3B) can be received and interpreted to create the ultrasound image 200. In general, the receive beam 310 will overlap with and be aligned with the transmit beam 310. Because of dynamic receive beamforming in traditional ultrasound, the receive beam may be fairly narrow for all depths. A transmit beam on the other hand has a fixed focal depth, and will be hourglass shaped with the narrowest portion having a width that matches the receive beam width.

**Figure 4B** is a schematic representation of an example lung ultrasound image 200. Visible are the ultrasound probe 10, body surface 210, pleural line 220, region of interest 240, depth selector 250, anatomical feature 260, and ultrasound receive beam 410.

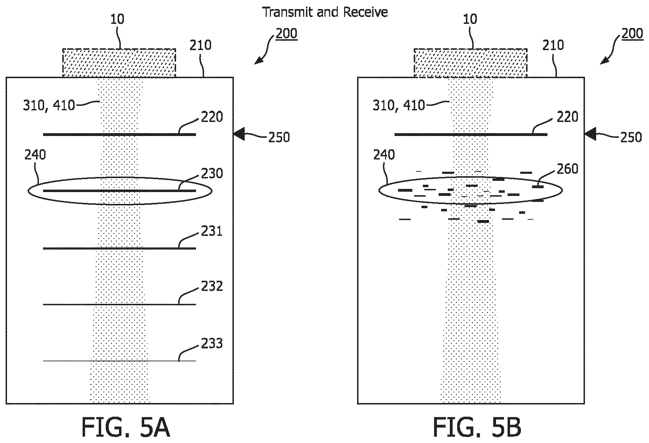

**Figure 5A** is a schematic representation of an example lung ultrasound image 200. Visible are the ultrasound probe 10, body surface 210, pleural line 220, region of interest 240, depth selector 250, A-line artifacts 230, 231, 232, and 233, and transmit beam 310. Also visible is a receive beam 410, which has been modified according to embodiments of the present disclosure, such that instead of a cone shape as seen in Figures 4A and 4B, the receive beam 410 has an hourglass shape generally similar to (and in some embodiments, coincident or overlapping with) the shape of the transmit beam 310, such that its narrowest portion occurs at or near the focal depth of the transmit beam 310 (e.g., desired focal depth D as shown for example in Figure 2A, which is the depth indicated by depth selector 250). Thus, the modified receive beam 410 has a focal depth similar to the focal depth of the transmit beam 310, which may have the effect of enhancing features at that focal depth, and de-enhancing features at depths different from the focal depth (e.g., features becoming dimmer and blurrier to more distant they are from the focal depth).

**Figure 5B** is a schematic representation of an example lung ultrasound image 200, in accordance with at least one embodiment of the present disclosure. Visible are the ultrasound probe 10, body surface 210, pleural line 220, region of interest 240, depth selector 250, anatomical feature 260, transmit beam 310, and modified receive beam 410.

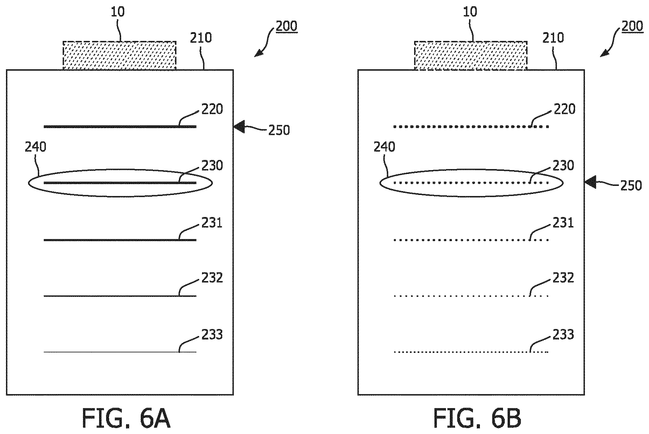

**Figure 6A** is a schematic representation of an example lung ultrasound image 200. Visible are the ultrasound probe 10, body surface 210, pleural line 220, region of interest 240, depth selector 250, and A-line artifacts 230, 231, 232, and 233. In the example shown in Figure 6A, the depth selector 250 is set to the depth of the pleural line 220, such that the focal depth of both the transmit beam and the modified receive beam may be set to this depth. This may for example have the effect of enhancing the appearance (e.g., brightness, sharpness, resolution, etc.) of the pleural line 220 in the ultrasound image 200, which may be of diagnostic value in evaluating the health of the pleural line. However, setting the focal depth to the pleural depth may also enhance the appearance of A-line artifacts 230, 231, 232, and 233. In some cases, this may be of diagnostic value, such that a care provider may wish to display the resulting ultrasound image 200 (e.g., on display 30 of Figure 1). In other cases, although the enhancement of the pleural line 220 may be desirable, enhancement of the A-line artifacts 230, 231, 232, and 233 will obscure real lung anatomy below the pleural line 220.

**Figure 6B** is a schematic representation of an example lung ultrasound image 200. Visible are the ultrasound probe 10, body surface 210, pleural line 220, region of interest 240, depth selector 250, and A-line artifacts 230, 231, 232, and 233. In the example shown in Figure 6B, the depth selector 250 is set to the depth of the region of interest 240, such that the focal depth of both the transmit beam and the modified receive beam may be set to this depth. This may for example have the effect of decreasing the appearance (e.g., brightness, sharpness, resolution, etc.) of A-line artifacts 230, 231, 232, and 233, which may improve a technician's or caregiver's ability to obtain clear images of lung anatomy below the pleural line. However, setting the focal depth to the depth of the region of interest may also decrease the appearance of the pleural line 220 itself. In some cases, this may be of little consequence, such that a care provider may still wish to display the resulting ultrasound image 200 (e.g., on display 30 of Figure 1). In other cases, although the decreased appearance of the pleural line 220 may be undesirable.

In some cases, when the appearance of an imaging artifact (E.g., A-line artifact 230) in the region of interest is decreased, it becomes clear that there is actually no clinically relevant anatomy within the region of interest, but rather the imaging artifact was simply mistaken for anatomy. Thus, a comparison between the ultrasound images of Figures 6A and 6B may be diagnostically useful.

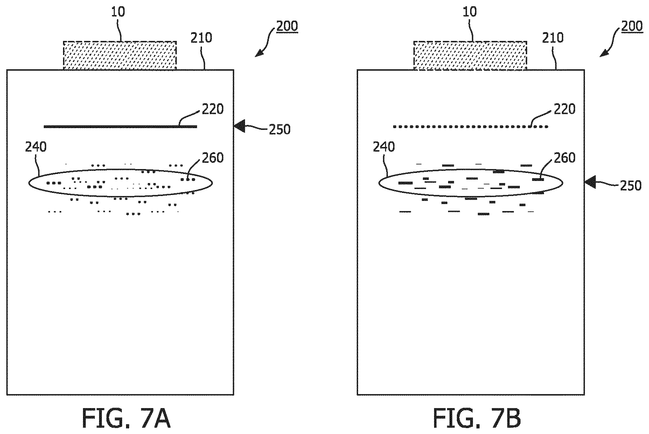

**Figure 7A** is a schematic representation of an example lung ultrasound image 200. Visible are the ultrasound probe 10, body surface 210, pleural line 220, region of interest 240, depth selector 250, and anatomical feature 260. In the example shown in Figure 7A, the depth selector 250 is set to the depth of the pleural line 220, such that the focal depth of both the transmit beam and the modified receive beam may be set to this depth. This may for example have the effect of enhancing the appearance (e.g., brightness, sharpness, resolution, etc.) of the pleural line 220 in the ultrasound image 200, which may be of diagnostic value in evaluating the health of the pleural line 220. However, setting the focal depth to the pleural depth may decrease the appearance (e.g., brightness, sharpness, resolution, etc.) of the anatomical feature 260, such that the ultrasound image 200 is less useful for diagnosing the health implications of the anatomical feature 260.

**Figure 7B** is a schematic representation of an example lung ultrasound image 200. Visible are the ultrasound probe 10, body surface 210, pleural line 220, region of interest 240, depth selector 250, and anatomical feature 260. In the example shown in Figure 7B, the depth selector 250 is set to the depth of the region of interest 240, such that the focal depth of both the transmit beam and the modified receive beam may be set to this depth. This may for example have the effect of enhancing the appearance (e.g., brightness, sharpness, resolution, etc.) of the anatomical feature 260 in the ultrasound image 200, which may be of diagnostic value in evaluating the health implications of the anatomical feature 260. However, setting the focal depth to the depth of the region of interest may decrease the appearance (e.g., brightness, sharpness, resolution, etc.) of the pleural line 220. such that the ultrasound image 200 is less useful for diagnosing the health of the pleural line 220.

Thus, it may be helpful for the technician or caregiver to display two ultrasound images (for example, side-by-side) that have two different focal depths, in order to resolve the features of both the pleural line 220 and the anatomical feature 260 within the region of interest 240.

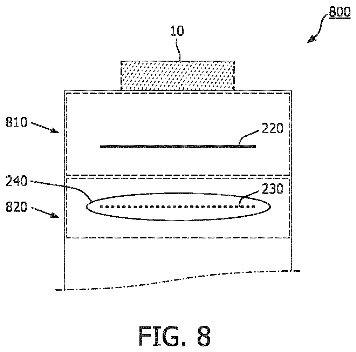

**Figure 8** is a schematic representation of an example combined lung ultrasound image 800. The combined image includes an upper image region 810 captured at a first focal depth (such as, for example, the pleural depth) and a lower image region 820, captured at a second focal depth (such as, for example, the depth of the region of interest 240). In the example shown in Figure 8, the upper image region 810 includes portions of an ultrasound image captured at a focal depth approximately equal to the pleural depth, as shown for example in Figure 6A, while the lower image region 820 includes portions of an ultrasound image captured at a focal depth approximately equal to the depth of the region of interest 240, as shown for example in Figure 6B. In an example, the upper image region 810 and the lower image region 820 are positioned edge-to-edge such that they appear to be portions of a single ultrasound image. This arrangement permits the combined ultrasound image 800 to include an enhanced pleural line 220, but a diminished A-line artifact 230, such that the A-line artifact 230 is less obscuring of any anatomy that may be present in the region of interest 240. In some cases, it may be desirable to include diminished anatomy and enhanced artifacts in the combined image, depending on the diagnostic needs of the clinician. In some cases, a feature of interest such as an A-line may be present over only a small lateral extent of the image (e.g., on the left or right side), while other laterally different locations (e.g., the opposite side) have an anatomical feature such as a consolidation (e.g., at different depth, or even at same depth). In such cases, smart blending as described herein could highlight both types of features of interest at the same time.

It should be understood that the combined image 800 can combine more than two image regions. For example, the combined image 800 may include three, four, or five image regions, each captured at a different focal depth according to embodiments of the present disclosure.

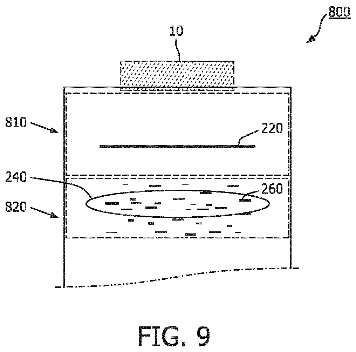

**Figure 9** is a schematic representation of an example combined lung ultrasound image 800. The combined image includes an upper image region 810 captured at a first focal depth (such as, for example, the pleural depth) and a lower image region 820, captured at a second focal depth (such as, for example, the depth of the region of interest 240). In the example shown in Figure 8, the upper image region 810 includes portions of an ultrasound image captured at a focal depth approximately equal to the pleural depth, as shown for example in Figure 6A, while the lower image region 820 includes portions of an ultrasound image captured at a focal depth approximately equal to the depth of the region of interest 240, as shown for example in Figure 7B. In an example, the upper image region 810 and the lower image region 820 are positioned edge-to-edge such that they appear to be portions of a single ultrasound image. This arrangement permits the combined ultrasound image 800 to include both an enhanced pleural line 220 and an enhanced anatomical feature 260, wherein the enhanced anatomical feature 260 is minimally obscured by A-line artifacts. In some cases, it may be desirable to include diminished anatomy and enhanced artifacts in the combined image, depending on the diagnostic needs of the clinician.

In some embodiments, forming the combined image may involve digitally blending the first image and the second image in a spatially varying manner, based on a determination of artifact versus true anatomy. For example, when comparative analysis of the two images indicates that there is an artifact at the ROI (e.g., if the feature of interest in the ROI is sharper or more pronounced in the first image), then the two images may be blended in such a way that the second image is weighted less strongly around the ROI, and the first image is weighted more strongly. Conversely, the blending algorithm may put more weight on the second image if the feature of interest is believed to be true anatomy (e.g., if the feature in the ROI is sharper or more pronounced in the second image). This output blending function can guide, for each region of the two images, in what ratio the images should be blended. For visually pleasing results, it may be desirable for the blending function to have spatial variations that are smooth. In addition, one could also add false color to features believed to be originating from artifacts. In some cases, the weighting may also be set by a user, for example, an operator of the ultrasound system 100 may set the weight of the first and/or second ultrasound image to be blended via a user interface 20.

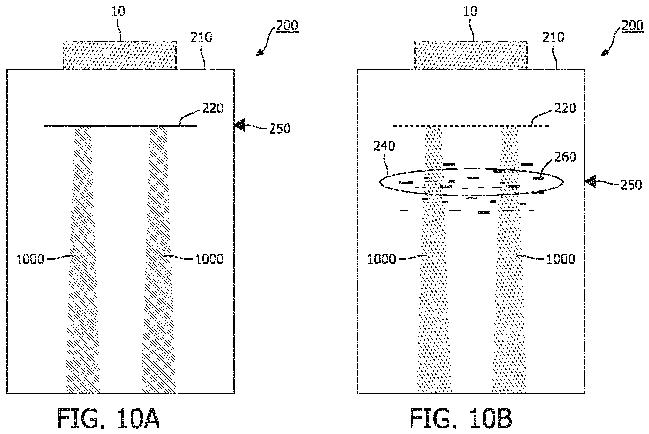

**Figure 10A** is a schematic representation of an example lung ultrasound image 200. Visible are the ultrasound probe 10, body surface 210, pleural line 220, region of interest 240, and depth selector 250. Also visible are two B-line artifacts 1000, which may for example be cone-shaped ultrasound "shadows" where the strength of the ultrasound transmit beam is decreased or increased.

B-line artifacts 1000 may be caused for example by shape, thickness, or density variations in an anatomical structure such as the pleural line 220. B-line artifacts 1000 (also called 'comet-tail' artifacts) may for example be vertical hyperechoic lines that extend posteriorly from the opposed pleura to the bottom of the screen or maximum imaging depth. The characteristics of the B-lines, such as the number, width, and/or intensity of the B-lines, may change as the patient breathes. In the example shown in Figure 10A, the focal depth has been set at least approximately to the pleural depth, such that the appearance of the pleural line 220 is enhanced in the ultrasound image 200. However, this may also have the effect of enhancing the B-line artifacts 1000, which may or may not be desirable for determining the health status of the patient. In many cases, it may be clinically desirable to see the B-lines clearly, for example by setting the transmit and receive depths to the depth of the pleural line as shown in Figure 10A.

**Figure 10B** is a schematic representation of an example lung ultrasound image 200. Visible are the ultrasound probe 10, body surface 210, pleural line 220, region of interest 240, depth selector 250, and B-line artifacts 1000. In the example shown in Figure 10B, the focal depth has been set below that of the pleural line 220, such as, for example the depth of an anatomical feature 260 within a region of interest. As a result, the appearance of the B-lines 1000 is diminished, which may be useful in resolving the anatomical feature 260 within the region of interest 240. However, this may also have the effect of decreasing the appearance of the pleural line 220, which may be undesirable. Thus, it may be desirable to produce a combined image including for example the enhanced pleural line 220 of Figure 10A and the enhanced anatomical feature of Figure 10B, as shown for example in Figure 9.

To develop a full clinical picture of the patient's lung health, it may be desirable to estimate, for each image location, if any visible features of interest are artifacts or true anatomy, and depending on that, use portions of each image to generate a combined image that enhances the desired features.

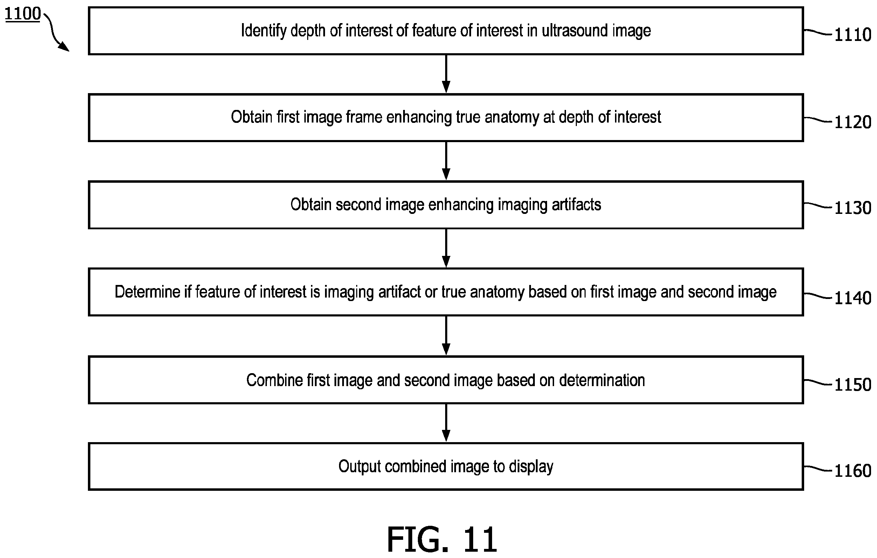

**Figure 11** shows a flow diagram of an example dual-focus ultrasound imaging method 1100. It is understood that the steps of method 1100 may be performed in a different order than shown in Figure 11, additional steps can be provided before, during, and after the steps, and/or some of the steps described can be replaced or eliminated in other embodiments. One or more of steps of the method 1100 can be carried by one or more devices and/or systems described herein, such as components of the ultrasound system 100 and/or processor circuit 1250.

In step 1110, the method 1100 includes, with an ultrasound system, capturing an initial ultrasound image (e.g., of a patient's lung), and identifying both the pleural line and a feature of interest in the initial ultrasound image (e.g., a region containing anatomy of interest, such as for example a suspected consolidation, pleural effusion, air bronchogram, fluid, pneumothorax, or other lung anomaly). In some embodiments, the pleural line and feature of interest may be identified automatically (e.g., through feature recognition by a machine learning algorithm or other artificial intelligence). In some embodiments, the pleural line and feature of interest may be identified at least partially with the assistance of a user (e.g., by means of the user touching, clicking or drawing on the ultrasound image via a user interface), or by a hybrid approach. Identifying the pleural line and the feature of interest includes identifying the respective depths of the pleural line and feature of interest, or depth of interest. In some embodiments, step 1110 includes outputting the initial ultrasound image to a display (e.g., display 30 of Figure 1).

In step 1120, the method 1100 includes obtaining a first ultrasound image that enhances true anatomical features at the depth of interest. This may be done for example by setting the focal depth of both the transmit beam and the receive beam of the ultrasound system to the depth of interest. This may have the effect of not only enhancing the appearance of any true anatomy located at the depth of interest, but also of decreasing the appearance of any imaging artifacts at or near the depth of interest. In some embodiments, step 1120 includes outputting the first ultrasound image to a display (e.g., display 30 of Figure 1).

In step 1130, the method 1100 includes obtaining a second ultrasound image that enhances image artifacts and/or patient anatomy responsible for the artifacts. Image artifacts may for example include A-line artifacts and B-line artifacts, and may be indicative of a condition of an anatomical feature such as the pleural line of the patient's lung. Thus, enhancing the image artifacts may be achieved for example by setting the focal depth of both the transmit beam and receive beam of the ultrasound system to the depth of the responsible anatomy, e.g., the pleural line. The second image may therefore include enhancement of the pleural line as well as of the image artifacts, and may include decreased or degraded views of any true anatomy located at the depth of interest. In some embodiments, step 1130 includes outputting the second ultrasound image to a display (e.g., display 30 of Figure 1), either by itself or along with (e.g., side-by-side with) the first ultrasound image.

In step 1140, the method 1100 includes determining, based on the first image and the second image, whether the feature of interest is an imaging artifact or true anatomy. Such a determination may be performed automatically (e.g., via image recognition by a machine learning algorithm or other artificial intelligence), or with the assistance of the user (e.g., by touching, drawing, or clicking on the screen, or by activating controls of the ultrasound system).

The output from step 1140 could for example be a binary classification for each region of the two images, or could take the form of a more gradual rating indicating to what degree a region appears to be artifact or true anatomy.

In step 1150, the method 1100 includes forming a combined image. The combined image may for example include a portion of the first image that includes the focal depth of the first image, and a portion of the second image that includes the focal depth of the second image, or digital blending of the two images based on a weighting algorithm for different regions of each image. Thus, for example, the combined image may include an enhanced pleural line as well as an enhanced view of the depth of interest, with minimal imaging artifacts. It may then be clearer, either to an image recognition algorithm or to a clinician, whether there is clinically relevant true anatomy at the depth of interest.

In other instances, the deeper part of the combined image may use the shallow (e.g., pleural line) focal depth, while the shallower regions of the image are generated with a deeper (e.g., ROI) focal depth. Such a combined image may help clarify whether features of interest are pleural line related artifacts at the depth of interest. In some cases, false color may also be introduced (either at this step or another step), such that, for example, if a feature in an ROI is determined to be an image artifact (e.g., caused by the pleural line), then the feature of interest is given a false color such as a red, blue, or green hue.

In some embodiments, step 1150 includes blending (e.g., digitally blending) the first image and the second image in a spatially varying manner, based on determination of artifact versus true anatomy. In a non-limiting example, when comparative analysis of the two images indicates that there is an artifact at a particular location, then the two images may be blended in such a way that the second image is weighted less strongly around that location, and the first image is weighted more strongly. Conversely, the blending algorithm may put more weight on the second image if the feature in that location is believed to be true anatomy.

This output blending function from step 1140 can guide, for each region of the two images, in what ratio the images should be blended. For visually pleasing results, it may be desirable for the blending function to have spatial variations that are smooth. Hence some preprocessing may be done on the blending function, to create smooth variations along its depth and optionally very gradual variations across the lateral direction.

In step 1160, the method 1100 includes outputting the combined image to a display (e.g., display 30 of Figure 1), either by itself or along with (e.g., side-by-side with) the first and/or second ultrasound image. In this manner, a user may also manually adjust the blending weights, and visually observe the results on the display 30 in real-time, i.e., while adjusting the weighting of the images to be blended.

This flow diagram is provided for exemplary purposes; a person of ordinary skill in the art will recognize myriad variations that nonetheless fall within the scope of the present disclosure. The logic of Figure 11 is shown as sequential. However, similar logic could be parallel, massively parallel, object oriented, real-time, event-driven, or otherwise, while accomplishing the same or similar functions.

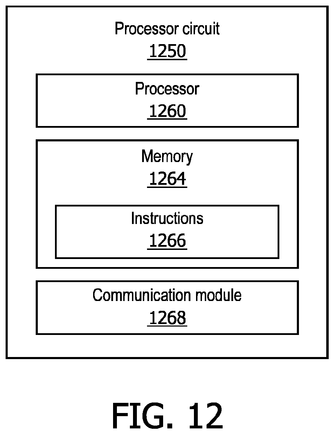

**Figure 12** is a schematic diagram of a processor circuit 1250, according to embodiments of the present disclosure. The processor circuit 1250 may be implemented in the ultrasound system 100, or other devices or workstations (e.g., third-party workstations, network routers, etc.), or on a cloud processor or other remote processing unit, as necessary to implement the methods described herein. As shown, the processor circuit 1250 may include a processor 1260, a memory 1264, and a communication module 1268. These elements may be in direct or indirect communication with each other, for example via one or more buses.

The processor 1260 may include a central processing unit (CPU), a digital signal processor (DSP), an ASIC, a controller, or any combination of general-purpose computing devices, reduced instruction set computing (RISC) devices, application-specific integrated circuits (ASICs), field programmable gate arrays (FPGAs), or other related logic devices, including mechanical and quantum computers. The processor 1260 may also comprise another hardware device, a firmware device, or any combination thereof configured to perform the operations described herein. The processor 1260 may also be implemented as a combination of computing devices, e.g., a combination of a DSP and a microprocessor, a plurality of microprocessors, one or more microprocessors in conjunction with a DSP core, or any other such configuration.

The memory 1264 may include a cache memory (e.g., a cache memory of the processor 1260), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an embodiment, the memory 1264 includes a non-transitory computer-readable medium. The memory 1264 may store instructions 1266. The instructions 1266 may include instructions that, when executed by the processor 1260, cause the processor 1260 to perform the operations described herein. Instructions 1266 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements.

The communication module 1268 can include any electronic circuitry and/or logic circuitry to facilitate direct or indirect communication of data between the processor circuit 1250, and other processors or devices. In that regard, the communication module 1268 can be an input/output (I/O) device. In some instances, the communication module 1268 facilitates direct or indirect communication between various elements of the processor circuit 1250 and/or the ultrasound system 100. The communication module 1268 may communicate within the processor circuit 1250 through numerous methods or protocols. Serial communication protocols may include but are not limited to US SPI, I²C, RS-232, RS-485, CAN, Ethernet, ARINC 429, MODBUS, MIL-STD-1553, or any other suitable method or protocol. Parallel protocols include but are not limited to ISA, ATA, SCSI, PCI, IEEE-488, IEEE-1284, and other suitable protocols. Where appropriate, serial and parallel communications may be bridged by a UART, USART, or another appropriate subsystem.

External communication (including but not limited to software updates, firmware updates, preset sharing between the processor and a central server, or readings from the ultrasound system 100) may be accomplished using any suitable wireless or wired communication technology, such as a cable interface such as a USB, micro USB, Lightning, or FireWire interface, Bluetooth, Wi-Fi, ZigBee, Li-Fi, or cellular data connections such as 2G/GSM, 3G/UMTS, 4G/LTE/WiMax, or 5G. For example, a Bluetooth Low Energy (BLE) radio can be used to establish connectivity with a cloud service, for transmission of data, and for receipt of software patches. The controller may be configured to communicate with a remote server, or a local device such as a laptop, tablet, or handheld device, or may include a display capable of showing status variables and other information. Information may also be transferred on physical media such as a USB flash drive or memory stick.

The computer program product may be software that is downloadable from a server, e.g. via the internet. Alternatively, the computer program product may be stored on a (non-transitory) medium like a USB stick or an optical storage medium (e.g. CD-ROM, DVD-ROM).

Accordingly, the logical operations making up the embodiments of the technology described herein are referred to variously as operations, steps, objects, elements, components, or modules. Furthermore, it should be understood that these may occur or be performed in any order, unless explicitly claimed otherwise or a specific order is inherently necessitated by the claim language.

In some implementations, there may be more than one region of interest or depth of interest. The ultrasound images described above may be acquired in any order. In some embodiments, multiple transducer arrays may be capable of acquiring multiple ultrasound images at multiple depths simultaneously. It should further be understood that the described technology may be employed in imaging of numerous different body systems, including but not limited to the stomach, intestines, trachea, esophagus, sinuses, vocal cords, and others. In some embodiments, images at different depths may be captured in an alternating fashion such that sequential combined images can be assembled in real time or near-real time.

All directional references e.g., upper, lower, left, right, lateral, back, bottom, above, below, vertical, and horizontal, are only used for identification purposes to aid the reader's understanding of the claimed subject matter, and do not create limitations, particularly as to the position, orientation, or use of the dual-focus ultrasound imaging system. Connection references, e.g., coupled and connected, are to be construed broadly and may include intermediate members between a collection of elements and relative movement between elements unless otherwise indicated. As such, connection references do not necessarily imply that two elements are directly connected and in fixed relation to each other. The term "or" shall be interpreted to mean "and/or" rather than "exclusive or." The word "comprising" does not exclude other elements or steps, and the indefinite article "a" or "an" does not exclude a plurality. Unless otherwise noted in the claims, stated values shall be interpreted as illustrative only and shall not be taken to be limiting.

The above specification, examples and data provide a complete description of the structure and use of exemplary embodiments of the dual-focus ultrasound imaging system as defined in the claims. Although various embodiments of the claimed subject matter have been described above with a certain degree of particularity, or with reference to one or more individual embodiments, those skilled in the art could make numerous alterations to the disclosed embodiments without departing from the scope of the claimed subject matter.

Still other embodiments are contemplated. It is intended that all matter contained in the above description and shown in the accompanying drawings shall be interpreted as illustrative only of particular embodiments and not limiting. Changes in detail or structure may be made within the subject matter as defined in the following claims.

## Claims

1. A system (100) for imaging anatomy of a patient, the system comprising:
a processor (28) configured to:
generate, using ultrasound imaging data received from an ultrasound probe (10), an initial image including an anatomical feature (220), an image artifact (230) caused by the anatomical feature, and a region of interest (240) disposed below the anatomical feature and obscured by the image artifact;
determine a depth of the anatomical feature and a depth of the region of interest;
generate, using ultrasound imaging data, a first image including the anatomical feature (220), the region of interest (240), and the image artifact (230), wherein a focal depth of a transmit beam (310) associated with the first image and a focal depth of a receive beam (410) associated with the first image are set to the depth of the anatomical feature (220), wherein an appearance of the anatomical feature (220) and the image artifact (230) in the first image is enhanced with respect to the initial image, and wherein an appearance of the region of interest (240) in the first image is decreased with respect to the initial image; and
generate, using ultrasound imaging data, a second image including the anatomical feature (220), the region of interest (240), and the image artifact (230), wherein the focal depth of the transmit beam (310) associated with the second image and the focal depth of the receive beam (410) associated with the second image are set to the depth of the region of interest (240), wherein the appearance of the anatomical feature (220) and the image artifact (230) in the second image is decreased with respect to the initial image, and wherein the appearance of the region of interest (240) in the second image is enhanced with respect to the initial image; and
provide, to a display (30), an output based on at least one of the first image or the second image.

2. The system of claim 1, wherein the output comprises the first image and the second image.

3. The system of any of the preceding claims, wherein the first image and the second image are displayed simultaneously.

4. The system of any of the preceding claims,
wherein the processor is further configured to generate a combined image comprising aspects of the first image and the second image,
wherein the output comprises the combined image.

5. The system of claim 4, wherein portions of the combined image are assigned a color based on whether the portions include the anatomical feature or the imaging artifact.

6. The system of any of the preceding claims, wherein the anatomy comprises a lung.

7. The system of claim 6, wherein the anatomical feature comprises a pleural line of the lung.

8. The system of claim 7, wherein the processor is further configured to automatically identify the pleural line.

9. The system of any of the preceding claims, wherein the processor is further configured to determine, based on the first image and the second image, whether the region of interest contains a feature of interest.

10. The system of claim 9, wherein the feature of interest comprises at least one of a consolidation, pleural effusion, air bronchogram, fluid, or pneumothorax.

11. The system of claim 9 or 10, wherein the processor is further configured to automatically identify the feature of interest.

12. The system of any of the preceding claims, wherein the image artifact comprises one or more A-line artifacts and/or one or more B-line artifacts.

13. The system of any of the preceding claims, further comprising a user interface device communicatively coupled to the processor, and wherein the processor is configured to:
receive a user input, from the user interface device, identifying the region of interest in the initial image.

14. A computer-implemented method for imaging anatomy of a patient, the method comprising:
generating an initial image using ultrasound imaging data, wherein the initial image includes an anatomical feature (220), an image artifact (230) caused by the anatomical feature, and a region of interest (240) disposed below the anatomical feature and obscured by the image artifact (230);
determining a depth of the anatomical feature (220) and a depth of the region of interest (240);
generating a first image using ultrasound imaging data, wherein the first image includes the anatomical feature (220), the region of interest (240), and the image artifact (230), wherein a focal depth of a transmit beam (310) associated with the first image and a focal depth of a receive beam (410) associated with the first image are set to the depth of the anatomical feature (220), wherein an appearance of the anatomical feature (220) and the image artifact (230) in the first image is enhanced with respect to the initial image, and wherein an appearance of the region of interest (240) in the first image is decreased with respect to the initial image;
generating a second image using ultrasound imaging data, wherein the second image includes the anatomical feature (220), the region of interest (240), and the image artifact (230), wherein the focal depth of the transmit beam (310) associated with the second image and the focal depth of the receive beam (410) associated with the second image are set to the depth of the region of interest (240), wherein the appearance of the anatomical feature (220) and the image artifact (230) in the second image is decreased with respect to the initial image, and wherein the appearance of the region of interest (240) in the second image is enhanced with respect to the initial image; and
providing an output based on at least one of the first image or the second image.

15. A computer program product comprising instructions for enabling a processor (20) to carry out the method of claim 14.

## Patentansprüche

1. System (100) zur Bildgebung der Anatomie eines Patienten, wobei das System Folgendes umfasst:
einen Prozessor (28), der konfiguriert ist, zum:
Erzeugen, unter Verwendung von Ultraschallbildgebungsdaten, die von einer Ultraschallsonde (10) empfangen werden, eines Ausgangsbildes, das ein anatomisches Merkmal (220), ein durch das anatomische Merkmal verursachtes Bildartefakt (230) und einen unterhalb des anatomischen Merkmals liegenden und durch das Bildartefakt verdeckten Bereich von Interesse (240) einschließt;
Bestimmen einer Tiefe des anatomischen Merkmals und einer Tiefe des Bereichs von Interesse;
Erzeugen, unter Verwendung von Ultraschallbildgebungsdaten, eines ersten Bildes, das das anatomische Merkmal (220), den Bereich von Interesse (240) und das Bildartefakt (230) einschließt, wobei die Fokustiefe eines Sendestrahls (310), das dem ersten Bild zugeordnet ist, und die Fokustiefe eines Empfangsstrahls (410), das dem ersten Bild zugeordnet ist, auf die Tiefe des anatomischen Merkmals (220) eingestellt sind, wobei das Erscheinungsbild des anatomischen Merkmals (220) und des Bildartefakts (230) im ersten Bild im Vergleich zum Ausgangsbild verbessert ist und wobei das Erscheinungsbild des Bereichs von Interesse (240) im ersten Bild im Vergleich zum Ausgangsbild verringert ist; und
Erzeugen, unter Verwendung von Ultraschallbildgebungsdaten, eines zweiten Bildes, das das anatomische Merkmal (220), den Bereich von Interesse (240) und das Bildartefakt (230) einschließt, wobei die Fokustiefe des Sendestrahls (310), das dem zweiten Bild zugeordnet ist, und die Fokustiefe des Empfangsstrahls (410), das dem zweiten Bild zugeordnet ist, auf die Tiefe des Bereichs von Interesse (240) eingestellt sind, wobei das Erscheinungsbild des anatomischen Merkmals (220) und des Bildartefakts (230) im zweiten Bild im Vergleich zum Ausgangsbild verringert ist und wobei das Erscheinungsbild des Bereichs von Interesse (240) im zweiten Bild im Vergleich zum Ausgangsbild verbessert ist; und
Bereitstellen, an eine Anzeige (30), einer Ausgabe, auf der Grundlage mindestens eines von dem ersten Bild oder dem zweiten Bild.

2. System nach Anspruch 1, wobei die Ausgabe das erste Bild und das zweite Bild umfasst.

3. System nach einem der vorstehenden Ansprüche, wobei das erste Bild und das zweite Bild gleichzeitig angezeigt werden.

4. System nach einem der vorstehenden Ansprüche,
wobei der Prozessor ferner so konfiguriert ist, dass er ein kombiniertes Bild erzeugt, das Aspekte des ersten Bildes und des zweiten Bildes umfasst,
wobei die Ausgabe das kombinierte Bild umfasst.

5. System nach Anspruch 4, wobei Teilen des kombinierten Bildes eine Farbe zugewiesen wird, je nachdem, ob die Teile das anatomische Merkmal oder das Bildartefakt einschließen.

6. System nach einem der vorstehenden Ansprüche, wobei die Anatomie eine Lunge umfasst.

7. System nach Anspruch 6, wobei das anatomische Merkmal eine Pleuralinie der Lunge umfasst.

8. System nach Anspruch 7, wobei der Prozessor ferner so konfiguriert ist, dass er die Pleuralinie automatisch identifiziert.

9. System nach einem der vorstehenden Ansprüche, wobei der Prozessor ferner so konfiguriert ist, dass er auf der Grundlage des ersten Bildes und des zweiten Bildes bestimmt, ob der Bereich von Interesse ein Merkmal von Interesse enthält.

10. System nach Anspruch 9, wobei das Merkmal von Interesse mindestens eines von einer Konsolidierung, einem Pleuraerguss, einem Luftbronchogramm, einem Fluid oder einem Pneumothorax umfasst.

11. System nach Anspruch 9 oder 10, wobei der Prozessor ferner so konfiguriert ist, dass er das Merkmal von Interesse automatisch identifiziert.

12. System nach einem der vorstehenden Ansprüche, wobei das Bildartefakt ein oder mehrere A-Linienartefakte und/oder ein oder mehrere B-Linienartefakte umfasst.

13. System nach einem der vorstehenden Ansprüche, ferner umfassend eine Benutzerschnittstellenvorrichtung, die mit dem Prozessor kommunikativ verbunden ist, und wobei der Prozessor konfiguriert ist, zum:
Empfangen, von der Benutzerschnittstellenvorrichtung, einer Benutzereingabe, die den Bereich von Interesse im Ausgangsbild identifiziert.

14. Computerimplementiertes Verfahren zur Bildgebung der Anatomie eines Patienten, wobei das Verfahren Folgendes umfasst:
Erzeugen eines Ausgangsbildes unter Verwendung von Ultraschallbildgebungsdaten, wobei das Ausgangsbild ein anatomisches Merkmal (220), ein durch das anatomische Merkmal verursachtes Bildartefakt (230) und einen unterhalb des anatomischen Merkmals liegenden und durch das Bildartefakt (230) verdeckten Bereich von Interesse (240) einschließt;
Bestimmen einer Tiefe des anatomischen Merkmals (220) und einer Tiefe des Bereichs von Interesse (240);
Erzeugen eines ersten Bildes unter Verwendung von Ultraschallbildgebungsdaten, wobei das erste Bild das anatomische Merkmal (220), den Bereich von Interesse (240) und das Bildartefakt (230) einschließt, wobei eine Fokustiefe eines Sendestrahls (310), der dem ersten Bild zugeordnet ist, und eine Fokustiefe eines Empfangsstrahls (410), der dem ersten Bild zugeordnet ist, auf die Tiefe des anatomischen Merkmals (220) eingestellt sind, wobei ein Erscheinungsbild des anatomischen Merkmals (220) und des Bildartefakts (230) im ersten Bild im Vergleich zum Ausgangsbild verbessert ist und wobei das Erscheinungsbild des Bereichs von Interesse (240) im ersten Bild im Vergleich zum Ausgangsbild verringert ist;
Erzeugen eines zweiten Bildes unter Verwendung von Ultraschallbildgebungsdaten, wobei das zweite Bild das anatomische Merkmal (220), den Bereich von Interesse (240) und das Bildartefakt (230) einschließt, wobei die Fokustiefe des Sendestrahls (310), der dem zweiten Bild zugeordnet ist, und die Fokustiefe des Empfangsstrahls (410), der dem zweiten Bild zugeordnet ist, auf die Tiefe des Bereichs von Interesse (240) eingestellt sind, wobei das Erscheinungsbild des anatomischen Merkmals (220) und des Bildartefakts (230) im zweiten Bild im Vergleich zum Ausgangsbild verringert ist und wobei das Erscheinungsbild des Bereichs von Interesse (240) im zweiten Bild im Vergleich zum Ausgangsbild verbessert ist; und
Bereitstellen einer Ausgabe, auf der Grundlage mindestens eines von dem ersten Bild oder dem zweiten Bild.

15. Computerprogrammprodukt, das Anweisungen umfasst, die es einem Prozessor (20) ermöglichen, das Verfahren nach Anspruch 14 auszuführen.

## Revendications

1. Système (100) pour l'imagerie de l'anatomie d'un patient, le système comprenant :
un processeur (28) configuré pour :
générer, en utilisant des données d'imagerie par ultrasons reçues à partir d'une sonde à ultrasons (10), une image initiale incluant une caractéristique anatomique (220), un artefact d'image (230) causé par la caractéristique anatomique et une région d'intérêt (240) disposée sous la caractéristique anatomique et masquée par l'artefact d'image ;
déterminer une profondeur de la caractéristique anatomique et une profondeur de la région d'intérêt ;
générer, en utilisant des données d'imagerie par ultrasons, une première image incluant la caractéristique anatomique (220), la région d'intérêt (240) et l'artefact d'image (230), dans lequel une profondeur focale d'un faisceau d'émission (310) associé à la première image et une profondeur focale d'un faisceau de réception (410) associé à la première image sont établies à la profondeur de la caractéristique anatomique (220), dans lequel une apparence de la caractéristique anatomique (220) et de l'artefact d'image (230) dans la première image est améliorée par rapport à l'image initiale, et dans lequel une apparence de la région d'intérêt (240) dans la première image est diminuée par rapport à l'image initiale ; et
générer, en utilisant des données d'imagerie par ultrasons, une seconde image incluant la caractéristique anatomique (220), la région d'intérêt (240) et l'artefact d'image (230), dans lequel la profondeur focale du faisceau d'émission (310) associé à la seconde image et la profondeur focale du faisceau de réception (410) associé à la seconde image sont établies à la profondeur de la région d'intérêt (240), dans lequel l'apparence de la caractéristique anatomique (220) et de l'artefact d'image (230) dans la seconde image est diminuée par rapport à l'image initiale, et dans lequel l'apparence de la région d'intérêt (240) dans la seconde image étant améliorée par rapport à l'image initiale ; et
fournir, à un dispositif d'affichage (30), une sortie sur la base d'au moins une parmi la première image ou la seconde image.

2. Système selon la revendication 1, dans lequel la sortie comprend la première image et la seconde image.

3. Système selon l'une quelconque des revendications précédentes, dans lequel la première image et la seconde image sont affichées simultanément.

4. Système selon l'une quelconque des revendications précédentes,
dans lequel le processeur est en outre configuré pour générer une image combinée comprenant des aspects de la première image et de la seconde image,
dans lequel la sortie comprend l'image combinée.

5. Système selon la revendication 4, dans lequel une couleur est attribuée à des parties de l'image combinée sur la base de la condition si les parties comprennent la caractéristique anatomique ou l'artefact d'imagerie.

6. Système selon l'une quelconque des revendications précédentes, dans lequel l'anatomie comprend un poumon.

7. Système selon la revendication 6, dans lequel la caractéristique anatomique comprend une ligne pleurale du poumon.

8. Système selon la revendication 7, dans lequel le processeur est en outre configuré pour identifier automatiquement la ligne pleurale.

9. Système selon l'une quelconque des revendications précédentes, dans lequel le processeur est en outre configuré pour déterminer, sur la base de la première image et de la seconde image, si la région d'intérêt contient une caractéristique d'intérêt.

10. Système selon la revendication 9, dans lequel la caractéristique d'intérêt comprend au moins un parmi une consolidation, un épanchement pleural, un bronchogramme aérien, un liquide ou un pneumothorax.

11. Système selon la revendication 9 ou 10, dans lequel le processeur est en outre configuré pour identifier automatiquement la caractéristique d'intérêt.

12. Système selon l'une quelconque des revendications précédentes, dans lequel l'artefact d'image comprend un ou plusieurs artefacts de ligne A et/ou un ou plusieurs artefacts de ligne B.

13. Système selon l'une quelconque des revendications précédentes, comprenant en outre un dispositif d'interface utilisateur couplé de manière communicative au processeur, et dans lequel le processeur est configuré pour :
recevoir une entrée utilisateur, à partir du dispositif d'interface utilisateur, identifiant la région d'intérêt dans l'image initiale.

14. Procédé mis en œuvre par ordinateur pour l'imagerie de l'anatomie d'une patient, le procédé comprenant :
la génération d'une image initiale en utilisant des données d'imagerie par ultrasons, dans lequel l'image initiale inclut une caractéristique anatomique (220), un artefact d'image (230) causé par la caractéristique anatomique et une région d'intérêt (240) disposée sous la caractéristique anatomique et masquée par l'artefact d'image (230) ;
la détermination d'une profondeur de la caractéristique anatomique (220) et d'une profondeur de la région d'intérêt (240) ;
la génération d'une première image en utilisant des données d'imagerie par ultrasons, dans lequel la première image inclut la caractéristique anatomique (220), la région d'intérêt (240) et l'artefact d'image (230), dans lequel une profondeur focale d'un faisceau d'émission (310) associé à la première image et une profondeur focale d'un faisceau de réception (410) associé à la première image sont établies à la profondeur de la caractéristique anatomique (220), dans lequel une apparence de la caractéristique anatomique (220) et de l'artefact d'image (230) dans la première image est améliorée par rapport à l'image initiale, et dans lequel une apparence de la région d'intérêt (240) dans la première image étant diminuée par rapport à l'image initiale ;
la génération d'une seconde image en utilisant des données d'imagerie par ultrasons, dans lequel la seconde image inclut la caractéristique anatomique (220), la région d'intérêt (240) et l'artefact d'image (230), dans lequel la profondeur focale du faisceau d'émission (310) associé à la seconde image et la profondeur focale du faisceau de réception (410) associé à la seconde image sont établies à la profondeur de la région d'intérêt (240), dans lequel l'apparence de la caractéristique anatomique (220) et de l'artefact d'image (230) dans la seconde image est diminuée par rapport à l'image initiale, et dans lequel l'apparence de la région d'intérêt (240) dans la seconde image est améliorée par rapport à l'image initiale ; et
la fourniture d'une sortie sur la base d'au moins une parmi la première image ou la seconde image.

15. Produit de programme informatique comprenant des instructions permettant à un processeur (20) de réaliser le procédé selon la revendication 14.
